(19) Europäisches Patentamt
European Patent Office
Office européen des brevets

(11) Veröffentlichungsnummer: **0 053 716**
**A1**

# (12) EUROPÄISCHE PATENTANMELDUNG

(21) Anmeldenummer: **81109207.1**

(22) Anmeldetag: **29.10.81**

(51) Int. Cl.³: **C 07 C 43/162**
C 07 C 41/16, A 61 K 7/46
//C11D3/50, C11D9/44

(30) Priorität: **05.12.80 DE 3045960**

(43) Veröffentlichungstag der Anmeldung:
**16.06.82 Patentblatt 82/24**

(84) Benannte Vertragsstaaten:
**CH FR GB IT LI NL**

(71) Anmelder: **Henkel Kommanditgesellschaft auf Aktien**
**Postfach 1100 Henkelstrasse 67**
**D-4000 Düsseldorf 1(DE)**

(72) Erfinder: **Schaper, Ulf-Armin, Dr.**
**Nixenstrasse 17**
**D-4000 Düsseldorf 13(DE)**

(72) Erfinder: **Bruns, Klaus, Dr.**
**Notburgaweg 6**
**D-4150 Krefeld-Traar(DE)**

## (54) Terpenether, deren Herstellung und Verwendung sowie diese enthaltende Riechstoffkompositionen.

(57) Die Ether des 3-(4-Methyl-3-cyclohexen-1-yl)-butanols (I) stellen wertvolle neue Riechstoffe mit kräftigen, haftfähigen Duftnoten dar.

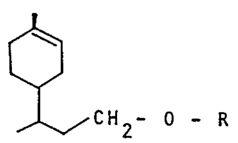
(1)

R = linearer, verzweigter, gesättigter oder ungesättigter Kohlenwasserstoffrest ($C_{1-4}$).

Für die Herstellung der neuen Terpenether (I) dient als Ausgangsprodukt 3-(4-Methyl-3-cyclohexen-1-yl)butyraldehyd. Dieser wird

a) mit einem komplexen Metallhydrid, z. B. Natriumborhydrid oder Lithiumaluminiumhydrid, zum 3-(4-Methyl-3-cyclohexen-1-yl)-butanol reduziert, welches
b) mit einem Lithiumalkyl oder einer Grignard-Verbindung zum Alkoholat umgesetzt wird, welches
c) mit einem Kohlenwasserstoffhalogenid (RX) oder einem Dialkylsulfat ($R_2SO_4$) umgesetzt wird.

Die so erhaltenen Riechstoffe können Parfümkompositionen stimulierende Geruchsnuancen verleihen. Die hierfür einzusetzenden Mengen liegen bei 1 - 5o Gew.-%.



COMPLETE DOCUMENT
Croydon Printing Company Ltd.

0053716

Henkel KGaA
ZR-FE/Patente

Düsseldorf, den o3. 12. 198o Dr. jg./Rs.

P a t e n t a n m e l d u n g

D 6288

"Terpenether, deren Herstellung und Verwendung sowie diese enthaltende Riechstoffkompositionen"

Gegenstand der Erfindung sind Ether des 3-(4-Methyl-3-cyclohexen-1-yl) butanols (beziehungsweise des 9 Hydroxymethyl-1-p-menthens), deren Herstellung und Verwendung als Riechstoffe und diese enthaltende Riechstoffkompositionen.

Aus der Reihe der natürlichen Terpene und der synthetischen Terpenabkömmlinge sind zahlreiche Verbindungen mit Riechstoffeigenschaften bekannt. Trotzdem ist es auf dem Gebiet der Riechstoffe bisher nicht möglich, für neue Verbindungen deren Brauchbarkeit als Duftstoff vorherzusagen oder gar gezielt Duftstoffe mit bestimmten Geruchsnoten zu synthetisieren. Die Riechstoffindustrie hat ständig Bedarf an interessanten und wertvollen Duftkörpern, die es ermöglichen Kompositionen mit neuen, überraschenden Duftnuancen zu schaffen, und die auf synthetischem Wege aus wohlverfügbaren Ausgangsstoffen leicht zugänglich sind.

Im Rahmen der sich daraus ergebenden Aufgabenstellung wurde gefunden, daß Ether des 3-(4-Methyl-3-cyclohexen-1-yl) butanols der allgemeinen Formel

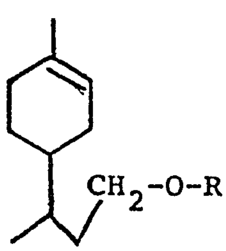

(I)

...

in welcher R eine lineare oder verzweigte, gesättigte oder ungesättigte Kohlenwasserstoffgruppe mit 1 - 4 Kohlenstoffatomen bedeutet, wertvolle neue Riechstoffe mit kräftigen, haftfähigen und daher parfümistisch verwertbaren Duftnoten darstellen. Besonders hervorzuheben sind der Methylether mit einer deutlichen Ho-Blätteröl- und Petitgrain-Note und der Ethylether mit einer fruchtig-süßen Duftnote.

Die Herstellung der erfindungsgemäßen neuen Riechstoffe erfolgt nach an sich bekannten Reaktionsverfahren der organischen Chemie, die in ihrer Folge und in Bezug auf die erfindungsgemäßen Verbindungen ein neues Herstellverfahren darstellen. Als Ausgangsmaterial für die Synthese dient Limonen, welches aus verschiedenen ätherischen Ölen leicht verfügbar ist. Dieses kann auf bekanntem Wege, durch Hydroformylierung (nach K. Kogami et al., Yakagaku 22, 316 (1973),(C.A. 79, 92400 n), in den 3-(4-Methyl-3-cyclohexen-1-yl)-butyraldehyd II überführt werden. Ausgehend von dem 3-(4-Methyl-3-cyclohexen-1-yl)-butyraldehyd II erfolgt die Herstellung der neuen Terpenether I durch

a) Reduktion mit einem komplexen Metallhydrid zum 3-(4-Methyl-3-cyclohexen-1-yl)-butanol III

b) Umsetzung des 3-(4-Methyl-3-cyclohexen-1-yl)-butanols mit einem Lithiumalkyl oder einer Grignard-Verbindung zum Alkoholat IV

c) Umsetzung des 3-(4-Methyl-3-cyclohexen-1-yl)-butylat IV mit einem Kohlenwasserstoffhalogenid

...

der allgemeinen Formel R-X oder einem Dialkylsulfat der allgemeinen Formel $R_2SO_4$,wobei R ein linearer, verzweigter, gesättigter oder ungesättigter Kohlenwasserstoffrest mit 1 - 4 Kohlenstoffatomen und X ein Halogenatom ist. Als Kohlenwasserstoffhalogenide verwendet man zweckmäßigerweise Jodide und Bromide.

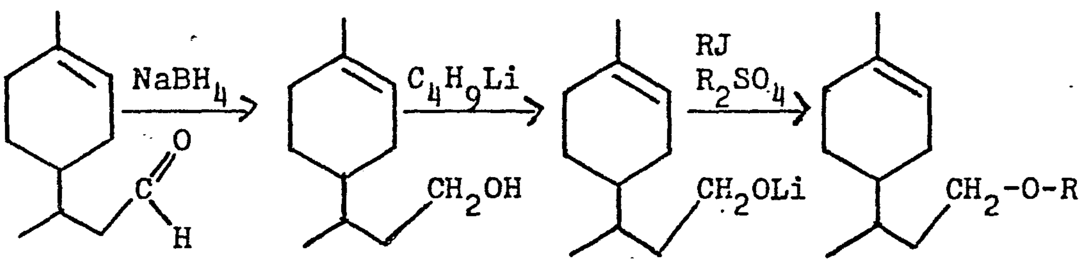


II III IV I

Als komplexe Metallhydride für die Reduktion des Aldehyds II eignen sich z. B. Natriumborhydrid und Lithiumaluminiumhydrid. Die Überführung des Alkohols III in das Alkoholat gelingt z.B. gut mit Butyllithium bei tiefer Temperatur in einem inerten Lösungsmittel, z.B. in n-Hexan. Ohne vorherige Isolation wird das Alkoholat IV im Sinne einer Williamson-Sythese weiter zum Ether I umgesetzt. Für die Herstellung des 3-(4-Methyl-3-cyclohexen-1-yl)-butanols III ist bereits ein anderes Herstellverfahren bekannt. Dieses geht ebenfalls aus von Limonen: Durch Umsetzung mit Paraformaldehyd erhält man das 9-Hydroxymethyl-1.8-(10)-p-menthadien, welches durch selektive katalytische Hydrierung in den einfach ungesättigten Alkohol III überführt werden kann (vergl. A.T. Blomquist, R.J. Himics, J. org. Chem. 33, 1156 (1968)).

...

Ein auf diesem Wege hergestelltes 3-(4-Methyl-3-cyclohexen-1-yl)-butanol III kann ebenfalls in der oben beschriebenen Weise weiter in die erfindungsgemäßen Ether I überführt werden.

Die erfindungsgemäßen neuen Terpenether der allgemeinen Formel I zeichnen sich neben ihrer jeweils eigentümlichen Duftnote durch eine gute Haftfestigkeit aus. Ein weiterer Vorteil ist eine sehr gute Kombinationsfähigkeit zu neuartigen überraschenden Duftstoffkompositionen, denen sie sowohl eine gute Haftfestigkeit als auch interessante und stimulierende Geruchsnuancen verleihen.

Die neuen Terpenether können mit anderen Riechstoffen in den verschiedensten Mengenverhältnissen zu neuen Riechstoffkompositionen gemischt werden. Im allgemeinen wird sich ihr Anteil in den Riechstoffkompositionen in den Mengen von 1 bis 50 Gewichtsprozent, bezogen auf die gesamte Komposition, bewegen. Solche Kompositionen können sowohl zur Parfümierung von kosmetischen Präparaten wie Duftwässern, Aerosolen, Cremes, Lotionen, Badepräparaten und Toilettenseifen als auch in der Extrait-Parfümierie verwendet werden. Sie können aber auch zur Geruchsverbesserung technischer Produkte, wie Wasch- und Reinigungsmittel, Weichspüler, Textilbehandlungsmittel und so weiter eingesetzt werden.

Zur Parfümierung der verschiedenen Produkte werden diesen die Kompositionen im allgemeinen in Konzentrationen von 0,05 bis 2 Gewichtsprozent, bezogen auf das gesamte Produkt, zugesetzt.

Die nachfolgenden Beispiele sollen den Gegenstand der Erfindung näher erläutern, ohne ihn jedoch hierauf zu beschränken:

...

# Beispiele

## Beispiel 1

Herstellung von 3-(4-Methyl-3-cyclohexen-1-yl)-butanol (III)

166 g (1 Mol) 3-(4-Methyl-3-cyclohexen-1-yl)-butanol (II) (hergestellt aus Limonen, Basis Citrusöl, durch Hydroformylierung) wurden bei ca. 30-40$^{o}$ C zu einer Mischung von 10,5 g Natriumborhydrid in 200 ml Isopropanol getropft. Nachdem eine halbe Stunde lang nachgerührt war, wurden 150 ml 10%ige Schwefelsäure zugegeben. Die obere Phase wurde abgetrennt und mit gesättigter Kochsalzlösung neutral gewaschen. Das Rohprodukt wurde über eine Vigreuxkolonne destilliert. Ausbeute 130 g (d.s. 77% der Theorie).

$n_D^{20}$: : 1,487

NMR ($CCl_4$) $\delta$: 0,85 ppm, d, 3H; 1,2-2,1 ppm, m, 13H;
3,6 ppm, t, 2H; 3,6 ppm, s,
1H austauschbar mit $D_2O$ 5,3 ppm, m, 1H

IR (Öl) $cm^{-1}$ : 3330, 3010, 2960, 2910, 2830, 1445, 1430, 1375, 1145, 1045

Die Reduktion des Aldehyds II kann auch mit Lithiumaluminiumhydrid in Ether durchgeführt werden.

...

Beispiel 2

3-(4-Methyl-3-cyclohexen-1-yl)-butyl-methylether I, $R=CH_3$)

17 g (0,1 Mol) 3-(4-Methyl-3-cyclohexen-1-yl)-butanol III wurden in 100 ml n-Hexan gelöst und bei -78° mit 32 g einer 20 %igen Lösung von Butyllithium in n-Hexan versetzt. Nach einer Stunde wurde auf 5° erwärmt und bei dieser Temperatur mit 12,6 g Dimethylsulfat versetzt. Danach wurde noch drei Stunden zum Sieden erhitzt. Das abgekühlte Gemisch wurde in Eiswasser gegossen, die wäßrige Phase wurde nach dem Abtrennen der Hexanphase mit Ether extrahiert. Die vereinigten organischen Phasen wurden neutral gewaschen, getrocknet und im Vakuum destilliert.

Ausbeute: 13 g 71 % d.Th.

$Kp_{0,01}$ 56 - 58°

$n^{20}_D$ 1,467

NMR ($CCl_4$) $\delta$: 0,85 ppm, d, 3H; 1,2 - 2,1 ppm, m, 13H; 3,2 ppm, s, 3H; 3,3 ppm, t, 2H; 5,3 ppm, m, 1H

IR (Öl) $cm^{-1}$ : 3050, 3010, 2970, 2920, 2880, 1445, 1435, 1380, 1120

Geruch: Ho-Blätteröl-, Petitgrain-Note

...

Beispiel 3

3-(4-Methyl-3-cyclohexen-1-yl)-butyl-ethylether (I, R=$-C_2H_5$)

Analog zum Beispiel 2 wurden 17 g Alkohol III mit 15,4 g Diethylsulfat verethert.

Ausbeute: 12,5 g 64 d.Th.

$Kp_{0,05}$ 64 - 66°

$n_D^{20}$ 1,47

NMR ($CCl_4$) $\delta$: 0,85 ppm, d, 3H; 1,1 ppm, t, 3H; 1,3-2,1 ppm, m, 13H; 3,3 ppm, q, 2H; 3,4 ppm, t, 2H; 5,3 ppm, m, 1H

IR (Öl) $cm^{-1}$ : 3050, 3010, 2970, 2925, 2880, 1450, 1435, 1380, 1120, 1110

Geruch: fruchtig, süß

Beispiel 4

Blumiger Komplex für Toilette-Seifen

| | | | |
|---|---|---|---|
| 3-(4-Methyl-3-cyclohexen-1-yl)-butylmethylether | 80 | Gew.-Teile | |
| Geraniol ex Citronellöl | 190 | " | " |
| Agruflor[R] (Henkel) | 150 | " | " |
| Phenylethylalkohol | 140 | " | " |
| Benzylacetat | 110 | " | " |

...

| | | |
|---|---|---|
| Florocyclene, PPL | 80 | Gew.-Teile |
| alpha-Isomethyljonon | 70 | " " |
| Tonalid | 40 | " " |
| Moschus-Keton | 30 | " " |
| alpha-Amylzimtaldehyd | 30 | " " |
| alpha-Hexylzimtaldehyd | 30 | " " |
| Lilial[R] (Givaudan) | 20 | " " |
| cis-Jasmon | 10 | " " |
| Nonalacton 10 % in DEP | 10 | " " |
| Indol 10 % in DEP | 10 | " " |
| | 1000 | Gew.-Teile |

(DEP = Diethylphthalat)

...

P a t e n t a n s p r ü c h e

1. Ether des 3-(4-Methyl-3-cyclohexen-1-yl)-butanols der allgemeinen Formel (I)

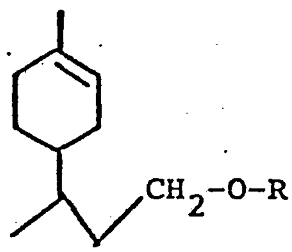

(I)

in welcher R ein linearer, verzweigter, gesättigter oder ungesättigter Kohlenwasserstoffrest mit 1-4 Kohlenstoffatomen ist.

2. Verfahren zur Herstellung von Ethern des 3-(4-Methyl-3-cyclohexen-1-yl-butanols der allgemeinen Formel I aus 3-(4-Methyl-3-cyclohexen-lyl)-butyraldehyd, gekennzeichnet durch die Reaktionsfolge.

a) Reduktion des 3-(4-Methyl-3-cyclohexen-1-yl)-butyraldehyd mit einem komplexen Metallhydrid zum 3-(4-Methyl-3-cyclohexen-1-yl)-butanol

b) Umsetzung des 3-(4-Methyl-3-cyclohexen-1-yl)-butanols mit einem Lithiumalkyl oder einer Grignard-Verbindung zum Alkoholat

c) Umsetzung des 3-(4-Methyl-3-cyclohexen-1-yl)-butylats mit einem Kohlenwasserstoffhalogenid der allgemeinen Formel RX oder einem Dialkylsulfat der allgemeinen Formel $R_2SO_4$, wobei R die in Anspruch 1 angegebene Bedeutung hat und X einen Halogenatom bedeutet.

3. Verwendung der Ether des 3-(4-Methyl-3-cyclohexen-1-yl)-butanols gemäß Anspruch 1 als Riechstoffe

...

4. Riechstoffkompostionen, dadurch gekennzeichnet, daß sie Ether des 3-(4-Methyl-3-cyclohexen-1-yl)-butanols gemäß Anspruch 1 in einer Menge von 1-50 Gewichtsprozent, bezogen auf die gesamte Riechstoffkompositionen, enthalten.

5. 3-(4-Methyl-3-cyclohexen-1-yl)-butyl-methylether

6. 3-(4-Methyl-3-cyclohexen-1-yl)-butyl-ethylether

...

0053716

Nummer der Anmeldung

Europäisches Patentamt

# EUROPÄISCHER RECHERCHENBERICHT

EP 81109207.1

| EINSCHLÄGIGE DOKUMENTE | | | KLASSIFIKATION DER ANMELDUNG (Int Cl.³) |
|---|---|---|---|
| Kategorie | Kennzeichnung des Dokuments mit Angabe, soweit erforderlich, der maßgeblichen Teile | betrifft Anspruch | |
| A | DE - B - 1 106 760 (IMPERIAL CHEMICAL)<br>* Patentanspruch 1 *<br>---- | 1 | C 07 C 43/162<br>C 07 C 41/16<br>A 61 K 7/46//<br>C 11 D 3/50<br>C 11 D 9/44 |

RECHERCHIERTE SACHGEBIETE (Int. Cl.³)

C 07 C 43/00
C 07 C 41/00

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 01-03-1982 | REIF |